# EUROPEAN PATENT SPECIFICATION

(11) **EP 3 545 778 B1**
(45) Date of publication and mention of the grant of the patent: **28.01.2026**
(21) Application number: 17873695.5
(22) Date of filing: 21.11.2017
(51) Int. Cl.: A24F 40/05, A24F 40/40, A24F 40/10

(54) **ATOMIZER COMPRISING AN INCLINED ULTRASONIC ATOMIZING SHEET STRUCTURE AND ELECTRONIC CIGARETTE**
ZERSTÄUBER MIT GENEIGTER ULTRASCHALLZERSTÄUBUNGSFOLIENSTRUKTUR UND ELEKTRONISCHE ZIGARETTE
ATOMISEUR AVEC STRUCTURE DE FEUILLE D'ATOMISATION ULTRASONORE INCLINÉE ET CIGARETTE ÉLECTRONIQUE

(30) Priority: 22.11.2016 CN 201611046072
(43) Date of publication of application: 02.10.2019
(73) Proprietor: China Tobacco Hunan Industrial Co., Ltd., Changsha, Hunan 410007 (CN)
(72) Inventor: LIU, Jianfu, Changsha Hunan 410007 (CN); ZHONG, Kejun, Changsha Hunan 410007 (CN); GUO, Xiaoyi, Changsha Hunan 410007 (CN); HUANG, Wei, Changsha Hunan 410007 (CN); YU, Hong, Changsha Hunan 410007 (CN); DAI, Yuangang, Changsha Hunan 410007 (CN); YIN, Xinqiang, Changsha Hunan 410007 (CN); YI, Jianhua, Changsha Hunan 410007 (CN); ZHOU, Yongquan, Changsha Hunan 410007 (CN)
(74) Representative: Alpspitz IP
(86) International application number: PCT/CN2017/112129
(87) International publication number: WO 2018/095312

(56) References cited:
- CN-A- 105 768 238
- CN-A- 105 795 527
- CN-A- 105 876 870
- CN-A- 105 876 873
- CN-A- 107 684 109
- CN-U- 204 377 937
- CN-U- 205 432 143
- CN-U- 206 197 036
- CN-U- 206 197 036
- KR-U- 20130 006 714
- US-A- 4 301 093

## Description

### Field of the Invention

The present invention relates to an atomizer comprising an inclined ultrasonic atomizing sheet structure and an electronic cigarette, and belongs to the field of electronic cigarettes

### Background of the Invention

CN 105 876 873 A discloses an atomizer. CN 105 795 527 A discloses an atomizer. Tobacco tar is heated by a heating wire in the conventional atomizer. The tobacco tar or liquid is guided onto the heating wire by tobacco tar guide cotton or fiber. When the temperature of the heating wire is too high, the tobacco tar guide material such as the tobacco tar guide cotton or fiber is likely to burn out to produce harmful substances to harm the health of a smoker, and the burnt flavour is easily produced to affect the smoking taste. In addition, part of the heat of the heating wire is absorbed by the tobacco tar guide material, resulting in heat transfer loss, high power consumption and low efficiency, and causing the atomizer to burn hot.

Chinese patent application for invention CN201610160216.3 disclosed an ultrasonic atomizer and an electronic cigarette, wherein the ultrasonic atomizer comprises an atomizing sheet and a liquid guide structure for guiding liquid onto the upper surface of the atomizing sheet; the liquid guide structure is communicated with a liquid storage cavity; the upper surface of the atomizing sheet is communicated with an airflow channel; and the atomizing sheet comprises a piezoelectric ceramic layer and an electric conductor for driving the piezoelectric ceramic layer to vibrate. The atomizing sheet of the present teachings does not need to be provided with micropores to spray the atomizing gas, thus avoiding the situation that the atomizing gas cannot be sprayed because large atomizing gas particles block the micropores, and better preventing the atomizer from leaking liquid.

However, in the disclosed Chinese patent application CN201610160216.3, the piezoelectric ceramic is placed horizontally, and after the atomizer is placed for a period of time, the atomization surface of the piezoelectric ceramic is full of tobacco tar, so that the piezoelectric ceramic starts atomizing of the tobacco tar slowly, even smoke cannot be produced by atomization, and at the same time, the tobacco tar leaks or is sucked. Thus, smoke is produced only after excess tobacco tar on the atomization surface of the piezoelectric ceramic is cleaned by disassembly, which brings inconvenience to use and poor experience and needs to be urgently improved.

### Summary of the Invention

The present teachings aim to provide an atomizer and an electronic cigarette. The atomizer can prevent the atomizing sheet from being soaked in tobacco tar such that no smoke is produced by atomization. Further, the present teachings can also avoid the phenomenon of leakage and suction of tobacco tar and the poor experience.

In order to achieve the above objective, an atomizer according to claim 1 is provided. An inclined ultrasonic atomizing sheet structure, comprising atomization cotton and an ultrasonic atomizing sheet; wherein the ultrasonic atomizing sheet is obliquely arranged, and two ends of the atomization cotton are used for connecting to tobacco tar in a tobacco tar cavity via tobacco tar guide cotton, or the two ends of the atomization cotton are directly provided in the tobacco tar cavity; and a lower surface of the atomization cotton is in contact with an atomization surface of the ultrasonic atomizing sheet.

Thus, the ultrasonic atomizing sheet is obliquely arranged relative to the horizontal plane when the electronic cigarette is used. The two ends of the atomization cotton are in contact with the tobacco tar guide cotton or directly stretch into the tobacco tar cavity to realize a tobacco tar guide function. When the atomizer is in operation, sufficient tobacco tar supply is ensured; when the atomizer is not in operation, the excess tobacco tar on the ultrasonic atomizing sheet is returned to the tobacco tar guide cotton or the tobacco tar cavity through the atomization cotton at the lower end of the inclined portion, so that the surface of the ultrasonic atomizing sheet is not soaked in the tobacco tar, and a sufficient amount of tobacco tar is supplied for atomization.

According to the embodiments of the present teachings, the present teachings may be further optimized. The following shows technical solutions formed after optimization: Preferably, the ultrasonic atomizing sheet is a piezoelectric ceramic driven by an electric conductor to ultrasonically vibrate.

Preferably, the ultrasonic atomizing sheet has an inclination angle of A, wherein 0° <A<90° , more preferably, 50°≤A≤75°. Tests have proved that the effect of the inclination angle of 50°-75° is preferred.

According to a first embodiment of the present teachings, the ultrasonic atomizing sheet is arranged in a mounting cavity of a housing, and the mounting cavity is obliquely arranged; preferably, the ultrasonic atomizing sheet is arranged in the mounting cavity through a silica gel sleeve, the bottom of the mounting cavity is provided with a penetration groove, and the lower part of the atomization cotton penetrates through the penetration groove and is connected to the tobacco tar guide cotton.

In order to lock the tobacco tar guide cotton on the housing, a locking ring fixedly connected with the housing is arranged at the upper part of the tobacco tar guide cotton, an air tube for communicating with a suction nozzle is formed in the center of the locking ring, an air passing slot is formed in the outer wall of the locking ring, and the air passing slot is used for forming an air inflow passage between the outside and the atomization cotton.

A gap is formed between the bottom of the air tube of the locking ring and the upper surface of the atomization cotton; preferably, the width of the gap is 0.2 to 5 mm; more preferably, the width of the gap is 0.5 to 2.5 mm. Thus, it can be ensured that most of airflow passes over the surface of the atomization cotton and carries the ultrasonically atomized smoke away.

In order to prevent a gap between the atomization cotton and the atomization surface of the ultrasonic atomizing sheet from affecting the atomization effect of tobacco tar, a pressing structure which maintains contact between the atomization cotton and the atomization surface of the ultrasonic atomizing sheet is arranged on the upper surface of the atomization cotton; preferably, the pressing structure is a pressing spring. The pressing spring can always ensure that the atomization cotton fits the atomization surface of the ultrasonic atomizing sheet.

According to another embodiment of the present teachings, the ultrasonic atomizing sheet is arranged at the top of an atomizing seat, and a silica gel seat is arranged on the upper surface of the ultrasonic atomizing sheet; an air outlet is formed in the center of the silica gel seat, and an air inlet is formed at the lower part of the silica gel seat; the air outlet is used for communicating with the suction nozzle, and the air inlet is used for forming an air inflow passage between the outside and the atomization cotton. Since the ultrasonic atomizing sheet is obliquely placed, the ultrasonic atomizing sheet in operation ejects the smoke formed by atomization of the tobacco tar to the inner wall of the air outlet, the inner wall collects and condenses large-particle smoke into smoke droplets, and the smoke droplets drop onto the atomization cotton along the inner wall and are re-atomized. During this process, small-particle smoke is sucked by the user to prevent the large-particle smoke from being sucked to affect the taste of the smoke.

Preferably, the bottom of the silica gel seat is provided with a ventilation groove which is used for communicating the air outlet with the air inflow passage.

In order to ensure that the atomization cotton can better deliver the tobacco tar to the atomization surface of the ultrasonic atomizing sheet, the two ends of the atomization cotton pass through the gap between the atomizing seat and the silica gel seat and stretch into the tobacco tar cavity.

Based on the same inventive concept, the present teachings further provide an atomizer, including a housing and a suction nozzle; wherein the inclined ultrasonic atomizing sheet structure is arranged in the housing;

Two ends of the atomization cotton are connected to the tobacco tar in the tobacco tar cavity through the tobacco tar guide cotton, or the two ends of the atomization cotton are directly arranged in the tobacco tar cavity.

According to a first embodiment of the present teachings, a locking ring fixedly connected with the housing is arranged at the upper part of the tobacco tar guide cotton; the housing and the locking ring are detachably and fixedly connected, and the housing and the suction nozzle are also detachably and fixedly connected; preferably, the internal thread of the housing is connected to the external thread of the locking ring, and the external thread of the housing is connected to the internal thread of the suction nozzle.

An air tube is formed in the center of the locking ring, and the air tube and the hollow passage of the suction nozzle form an air outflow passage; an air inlet slot is formed between the housing and the suction nozzle, an air passing slot is formed in the outer wall surface of the locking ring, and the air inlet slot and the air passing slot form an air inflow passage which communicates with the air outflow passage.

In order to facilitate the removal and cleaning or replacement of the suction nozzle, the housing and the suction nozzle are detachably and fixedly connected; preferably, the internal thread of the housing is connected to the external thread of the suction nozzle. The removal and installation are more convenient and rapid by means of threaded connection.

According to another embodiment of the present teachings, a silica gel seat and an atomizing seat are arranged in the housing, and the ultrasonic atomizing sheet and the atomization cotton are arranged between the silica gel seat and the atomizing seat; an air outlet is formed in the center of the silica gel seat, and an air inlet is formed at the lower part of the silica gel seat; the air outlet communicates with the suction nozzle to form an air outflow passage, and the air inlet and the ventilation groove at the bottom of the silica gel seat form an air inflow passage which communicates with the air outflow passage.

Based on the same inventive concept, the present teachings further provide an electronic cigarette according to claim 13. The electronic cigarette includes a battery assembly and the atomizer; wherein the battery assembly is used for driving the ultrasonic atomizing sheet to ultrasonically vibrate through a battery; and the ultrasonic atomizing sheet of the atomizer is obliquely arranged relative to the horizontal plane when the electronic cigarette is used.

In order to facilitate adding of tobacco tar, the atomizer is detachably and fixedly connected to the battery assembly, and preferably, the atomizer is magnetically connected to the battery assembly.

In order to prevent a battery cover from scalding hands when the atomizer is in operation, the battery assembly includes a battery cover, a large bracket and a battery; and a heat insulation space is formed in the large bracket. The heat insulation space improves the user experience.

Preferably, an upper fixing cover is arranged at the bottom of the housing, a lower fixing cover is arranged at the top of the battery cover, an upper electrode is mounted on the upper fixing cover, a lower electrode is mounted on the lower fixing cover, and the upper electrode is electrically connected with the lower electrode; a key is mounted on a side face of the battery cover, a PCB(Printed Circuit Board) is arranged inside the key, the battery is electrically connected with the PCB, and the PCB is in contact with the lower electrode when the PCB 24 is pressed by the key 23.

With the above structure, the inclined piezoelectric ceramic is placed in the electronic cigarette atomizer at an inclination angle of A, wherein 90° <A<0° , and since the angle is an inclination angle, the piezoelectric ceramic is at different heights on two sides, and the atomization cotton is arranged from the high side to the low side. Thus, when being guided, the tobacco tar passes through the atomization cotton to reach the surface of the piezoelectric ceramic for atomization. When the inclined atomization cotton sucks the tobacco tar to be saturated, no excess tobacco tar is produced to soak the atomization surface of the piezoelectric ceramic, so the problems that the piezoelectric ceramic soaked in the tobacco tar is started slowly and the amount of smoke is small are solved, and the user experience is improved.

Compared with the prior art, the present teachings have the following advantages:
1. An inclined ultrasonic atomizing sheet is arranged in the atomizer, and the ultrasonic atomizing sheet is a solid piezoelectric ceramic; the inclination angle is A, wherein 0° <A<90° , preferably 50° ≤A≤75° ; by setting A within this angle range, two ends of the atomization cotton are in contact with the tobacco tar guide cotton or directly stretch into the tobacco tar cavity to realize a tobacco tar guide function; when the atomizer is in operation, sufficient tobacco tar supply is ensured; when the atomizer is not in operation, excess tobacco tar on the ultrasonic atomizing sheet is returned to the tobacco tar guide cotton or the tobacco tar cavity through the atomization cotton at the lower end of the inclined portion, so that the surface of the piezoelectric ceramic is not soaked in the tobacco tar, and a sufficient amount of tobacco tar is supplied for atomization.
2. The tobacco tar is transferred to the atomization cotton through the tobacco tar guide cotton, and then the piezoelectric ceramic can atomize the tobacco tar in the atomization cotton to produce smoke, thus well controlling the flow rate of the tobacco tar to affect the atomization rate.
3. The atomization cotton is in contact with a part of the surface of the piezoelectric ceramic to improve the operating rate of the piezoelectric ceramic, thereby improving the user experience.
4. The atomizer is magnetically connected with the battery to facilitate disassembly.
5. The suction nozzle can be removed for cleaning.
6. A large bracket is arranged in the battery assembly, and a heat insulation space is formed in the large bracket, thus preventing the heat generated by the battery assembly from being transferred to the battery cover to scald hands when the electronic cigarette is in operation.

### Brief Description of the Drawings

Fig. 1 is a structural principle diagram according to Embodiment 1 of the present teachings;
Fig. 2 is an exploded schematic view of Fig. 1;
Fig. 3 is an exploded cross-sectional view of an atomizer in Fig. 1;
Fig. 4 is a three-dimensional view of Fig. 1;
Fig. 5 is a structural principle diagram according to Embodiment 2 of the present teachings;
Fig. 6 is an exploded schematic view of an atomizer in Fig. 5;
Fig. 7 is an exploded cross-sectional view of the atomizer in Fig. 5.

In the above figures:
1-atomizer; 2-battery assembly; 11-suction nozzle; 12-locking ring; 13-pressing spring; 14-atomization cotton; 15-silica gel sleeve; 16-piezoelectric ceramic; 17-tobacco tar guide cotton; 18-housing; 19-upper fixing cover: 20-upper electrode; 21-lower fixing cover; 22-lower electrode; 23-key; 24-PCB; 25-large bracket; 26-battery cover; 27-battery; 28-charging assembly; 29-heat insulation space: 30-air inlet slot: 31-air groove; 32-air passing slot; 33-air port; 34-mounting cavity; 35-penetration groove; 36-connecting electrode; 37-atomizing seat; 38-silica gel seat; 39-air outlet; 40-air outlet; 41-support pillar: 42-ventilation groove.

### Detailed Description of the Embodiments

The present teachings will be described in detail below with reference to the accompanying drawings in combination with embodiments. It should be noted that the embodiments in the present teachings and the features in the embodiments can be combined with each other without conflicts. For ease of narrative, the terms "upper", "lower", "left" and "right" described below are only consistent with the upper, lower, left and right directions of the drawings, and do not limit the structure.

### Embodiment 1

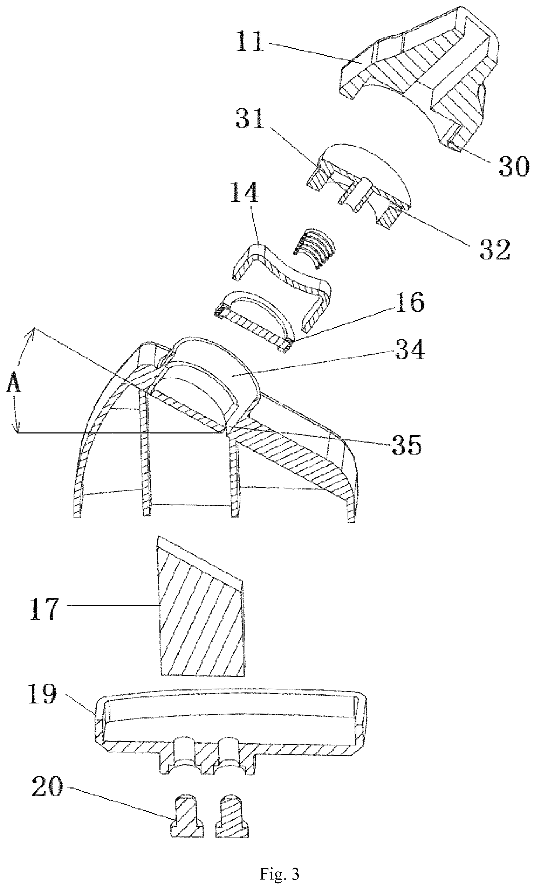

An inclined ultrasonic atomizing sheet structure, as shown in Fig. 3, includes atomization cotton 14, a piezoelectric ceramic 16, and tobacco tar guide cotton 17. The lower end of the tobacco tar guide cotton 17 is immersed in a tobacco tar cavity, and two ends of the atomization cotton 14 are in contact with the tobacco tar guide cotton 17, so that the tobacco tar is transferred to the atomization cotton 14 through the tobacco tar guide cotton 17, and then the piezoelectric ceramic 16 can atomize the tobacco tar in the atomization cotton 14 to produce smoke, thus well controlling the flow rate of the tobacco tar to affect the atomization rate.

The atomization cotton 14 of the present teachings is pressed on an atomization surface of the piezoelectric ceramic 16 through a pressing spring 13, thus avoiding poor contact between the atomization cotton 14 and the piezoelectric ceramic 16 due to vibration of the piezoelectric ceramic 16 in operation of the atomizer, thus the atomization effect or life of the piezoelectric ceramic 16 cannot be affected.

The piezoelectric ceramic 16 of the present teachings is a solid piezoelectric ceramic, which is driven by an electric conductor to form an ultrasonic atomizing sheet. The piezoelectric ceramic 16 of the present teachings is obliquely arranged at an inclination angle of A, preferably, 0° <A<90° , and more preferably, 50° ≤A≤75° . Within this angle range, two ends of the atomization cotton are in contact with the tobacco tar guide cotton to realize a tobacco tar guide function. When the atomizer is in operation, sufficient tobacco tar supply is ensured. When the atomizer is not in operation, excess tobacco tar on the piezoelectric ceramic is returned to the tobacco tar guide cotton through the atomization cotton at the lower end of the inclined portion, so that the surface of the piezoelectric ceramic is not soaked in the tobacco tar, and a sufficient amount of tobacco tar is supplied for atomization.

A piezoelectric ceramic mounting cavity 34 for placing the piezoelectric ceramic 16 in a housing 18 is also arranged obliquely. A penetration groove 35 is formed all around the bottom of the piezoelectric ceramic mounting cavity 34, and the atomization cotton 14 penetrates through the penetration groove 35 and is in contact with the tobacco tar guide cotton 17 to supply enough tobacco tar for the ultrasonic atomizing sheet.

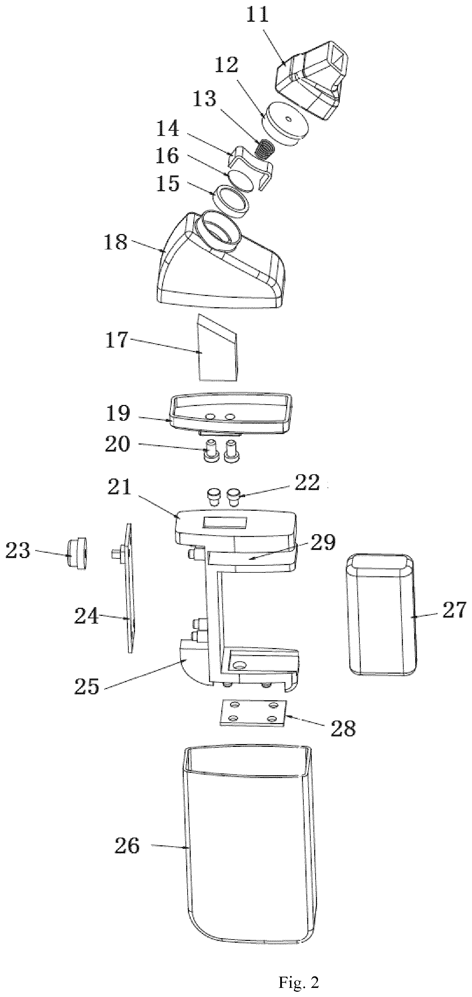

The atomization cotton of the present teachings is in contact with a part of the surface of the piezoelectric ceramic to improve the operating rate of the piezoelectric ceramic, thereby improving the user experience. An atomizer, as shown in Fig. 2 and Fig. 3, includes a suction nozzle 11 and a housing 18. A tobacco tar cavity is formed in the housing 18. The tobacco tar in the tobacco tar cavity enters the atomization cotton 14 through the tobacco tar guide cotton 17, the atomization cotton 14 delivers the tobacco tar to the atomization surface of the piezoelectric ceramic being as an ultrasonic atomizing sheet for atomization, and the atomized smoke is sucked by a smoker through the air outflow passage of the suction nozzle.

The internal thread of the housing 18 is connected with a locking ring 12, and the external thread of the housing 18 is connected with the suction nozzle 11. The suction nozzle 11 of the present teachings can be removed for cleaning.

An air tube 31 communicated with a suction passage is arranged in the center of the locking ring 12, an air passing slot 32 is formed in a side wall of the locking ring 12, an air inlet slot 30 is formed between the suction nozzle 11 and the housing 18, and the air inlet slot 30 is communicated with the air passing slot 32.

The air tube 31 of the present teachings is not in contact with the surface of the atomization cotton 14, and the distance between the air tube 31 and the atomization cotton 14 is preferably 0.2 to 5 mm, ensuring that most of airflow passes through the surface of the atomization cotton 14 and carries the ultrasonically atomized smoke away.

The lower end of the tobacco tar guide cotton 17 of the present teachings is in contact with the tobacco tar in the tobacco tar cavity, so that the tobacco tar can be absorbed by the tobacco tar guide cotton 17.

In the present teachings, the piezoelectric ceramic 16 is obliquely arranged, preferably at an inclination angle of more than or equal to 50° and less than or equal to 75° relative to the horizontal plane. Within this angle range, the two ends of the atomization cotton 14 are in contact with the tobacco tar guide cotton 17 to realize a tobacco tar guide function. When the atomizer 1 is in operation, sufficient tobacco tar supply is ensured. When the atomizer 1 is not in operation, excess tobacco tar on the piezoelectric ceramic 16 is returned to the tobacco tar guide cotton 17 through the atomization cotton 14 at the lower end of the inclined portion, thus preventing the piezoelectric ceramic 16 from being soaked in the tobacco tar.

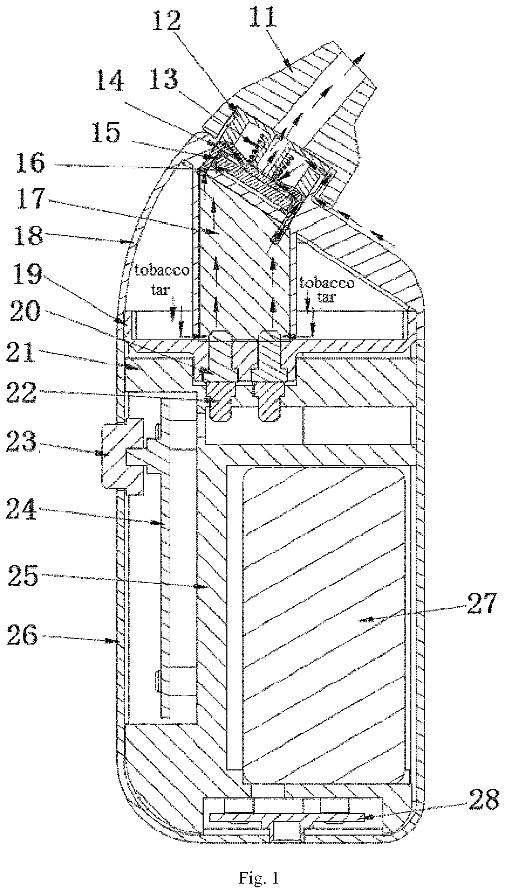

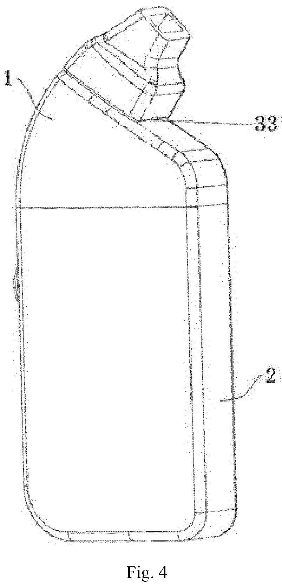

An electronic cigarette, as shown in Fig. 1 and Fig. 4, includes the above atomizer 1 and a battery assembly 2, wherein the atomizer 1 is magnetically connected with the battery assembly 2 to facilitate disassembly.

The battery assembly of the present teachings includes a battery cover 26, a large bracket 25, and a battery 27. A heat insulation space 29 is formed in the large bracket 25 to prevent the heat generated by the battery assembly 2 from being transferred to the battery cover 26 to scald hands when the electronic cigarette is in operation.

An upper fixing cover 19 is arranged at the bottom of the housing 18, a lower fixing cover 21 is arranged at the top of the battery cover 26, an upper electrode 20 is mounted on the upper fixing cover 19, a lower electrode 22 is mounted on the lower fixing cover 21, and the upper electrode 20 is electrically connected with the lower electrode 22. A key 23 is mounted on a side face of the battery cover 26, a PCB(Printed Circuit Board) 24 is arranged inside the key 23, the battery 27 is electrically connected with the PCB 24, and the PCB 24 is in contact with the lower electrode 22 when the PCB 24 is pressed by the key 23, so that the battery 27 supplies power to the piezoelectric ceramic being as an ultrasonic atomizing sheet for ultrasonic atomization.

A charging assembly 28 for charging the battery 27 is mounted at the bottom of the battery cover 26.

### Embodiment 2

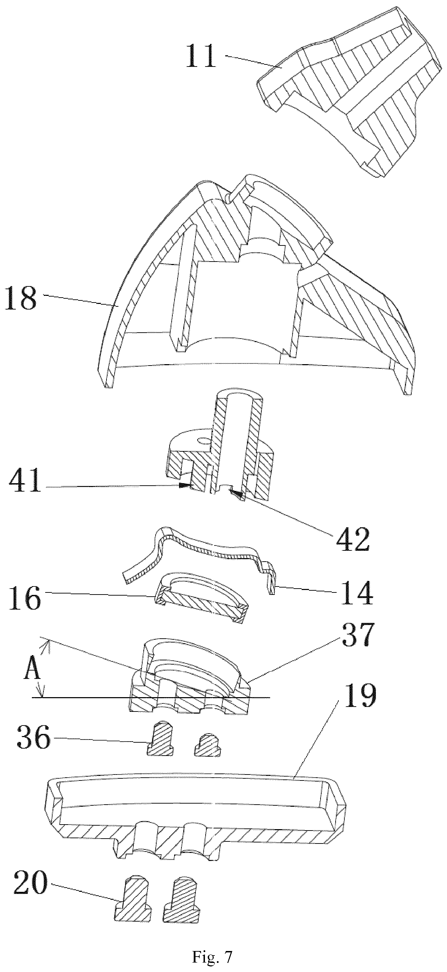

An inclined ultrasonic atomizing sheet structure, as shown in Fig. 7, includes atomization cotton 14 and a piezoelectric ceramic 16. Two ends of the atomization cotton 14 are directly immersed in the tobacco tar cavity, the tobacco tar is transferred to the piezoelectric ceramic 16 by the atomization cotton 14, and the tobacco tar in the atomization cotton 14 is atomized to produce smoke, thus well controlling the flow rate of the tobacco tar to affect the atomization rate.

The atomization cotton 14 of the present teachings is pressed on an atomization surface of the piezoelectric ceramic 16 through a silica gel seat 13, thus avoiding poor contact between the atomization cotton 14 and the piezoelectric ceramic 16 due to vibration of the piezoelectric ceramic 16 in operation of the atomizer 1.

The piezoelectric ceramic 16 of the present embodiment is similar to the ultrasonic atomizing sheet in Embodiment 1, and is driven by an electric conductor to form an ultrasonic atomizing sheet to atomize the tobacco tar. The piezoelectric ceramic 16 of the present teachings is obliquely arranged at an inclination angle of A. Preferably, 0° <A<90° , and more preferably, 50° ≤A≤75° . Within this angle range, two ends of the atomization cotton are directly immersed in a tobacco tar cavity to realize a tar guide function. When the atomizer is in operation, sufficient tar supply is ensured. When the atomizer is not in operation, excess tobacco tar on the piezoelectric ceramic is returned to the tobacco tar cavity through the atomization cotton at the lower end of the inclined portion, so that the tobacco tar on the atomization cotton is saturated, the surface of the piezoelectric ceramic does not accumulate the tobacco tar or is not soaked in the tobacco tar, and a sufficient amount of tobacco tar is supplied for atomization.

An air outlet 39 communicated with a suction passage and an air inlet 40 communicated with the atomization surface of the atomization cotton 14 are formed in the center of the silica gel seat 13 of the present embodiment, and the air inlet 40 is communicated with the air outlet 39 through a ventilation groove 42. Thus, the silica gel seat 13 is provided with an air inlet 40, and the airflow enters the atomization surface of the piezoelectric ceramic 16 through the air inlet 40, and then the smoke is discharged from the air outlet of the silica gel seat 40.

An atomizing seat 37 is mounted in the housing 18. The atomizing seat 37 is provided with a piezoelectric ceramic mounting cavity 34 for placing the piezoelectric ceramic 16. The atomization cotton 14 passes through the gap between the atomizing seat 37 and the silica gel seat 13, and the two ends of the atomization cotton 14 are in contact with the tobacco tar in the tobacco tar cavity, so that a sufficient amount of tobacco tar can be supplied to the ultrasonic atomizing sheet. The atomizing seat 37 is provided with an inclined cavity for placing the piezoelectric ceramic 16 at an inclination angle of A, 0° <A<90° , preferably 50° ≤A≤75° . Within this angle range, the two ends of the atomization cotton are directly immersed in the tobacco tar cavity to realize a tobacco tar guide function. When the atomizer is in operation, sufficient tobacco tar supply is ensured. When the atomizer is not in operation, excess tobacco tar on the piezoelectric ceramic is returned to the tobacco tar cavity through the atomization cotton at the lower end of the inclined portion, thus preventing the piezoelectric ceramic from being soaked in the tobacco tar.

The atomization cotton 14 of the present teachings is in contact with a part of the surface of the piezoelectric ceramic to improve the operating rate of the piezoelectric ceramic, thereby improving the user experience. The top of the atomization cotton 14 is pressed by a support pillar 41 arranged at the lower part of the silica gel seat 13, thereby preventing the atomization cotton 14 from being separated from the piezoelectric ceramic 16 to affect the atomization effect. The support pillar 41 abuts against the atomization cotton 14. When the piezoelectric ceramic 16 is in operation, the atomization cotton 14 is kept in contact with the piezoelectric ceramic 16, thereby increasing the atomization effect, and preventing the atomization cotton from being sucked up by smoking force to separate from the piezoelectric ceramic.

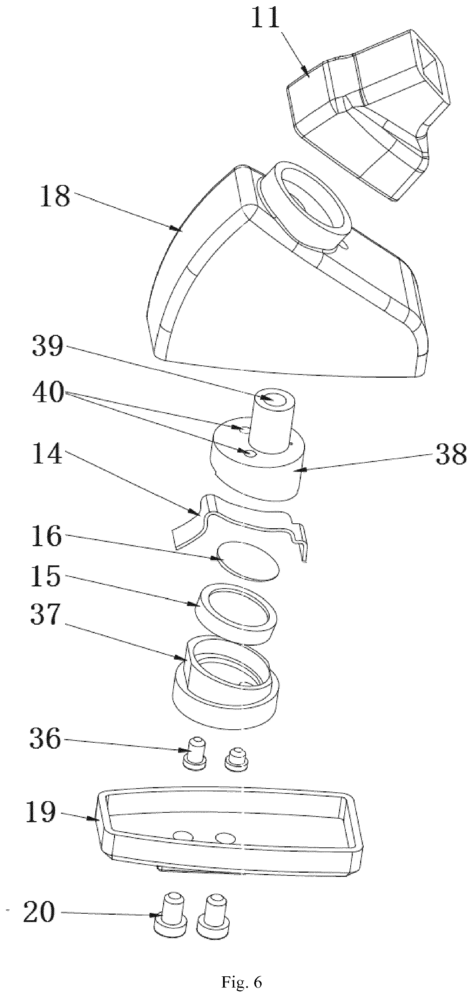

An atomizer, as shown in Figs. 6 and 7, includes a suction nozzle 11 and a housing 18. A tobacco tar cavity is formed in the housing 18. The tobacco tar in the tobacco tar cavity enters the atomization cotton 14, the atomization cotton 14 delivers the tobacco tar to the atomization surface of the piezoelectric ceramic being as an ultrasonic atomizing sheet for atomization, and the atomized smoke is sucked by a smoker through the air outflow passage of the suction nozzle.

The external thread of the housing 18 is connected with the suction nozzle 11. The suction nozzle 11 of the present teachings can be removed for cleaning.

A side wall of the housing 18 is provided with an air inlet slot 30 which communicates with an air inlet of the silica gel seat 13.

Since the piezoelectric ceramic 14 of the present teachings is placed obliquely, the smoke ejected when the piezoelectric ceramic 14 atomizes the tobacco tar is jetted onto the inner wall of the air outlet 39, so that the smoke droplets formed by the large-particle smoke drop onto the atomization cotton 14 along the inner wall and are recovered for re-atomization, and the smoke jetted from the piezoelectric ceramic 14 is prevented from directly impacting the user's mouth to produce bad experience such as tongue spiciness.

In addition, the two ends of the atomization cotton 14 of the present teachings directly stretch into the tobacco tar cavity to directly contact the tobacco tar, so that the tobacco tar guide speed is higher when atomizing, and the atomization effect is better. At the same time, the piezoelectric ceramic 16 is placed obliquely, so the tobacco tar is not accumulated, and the atomization starting rate is improved.

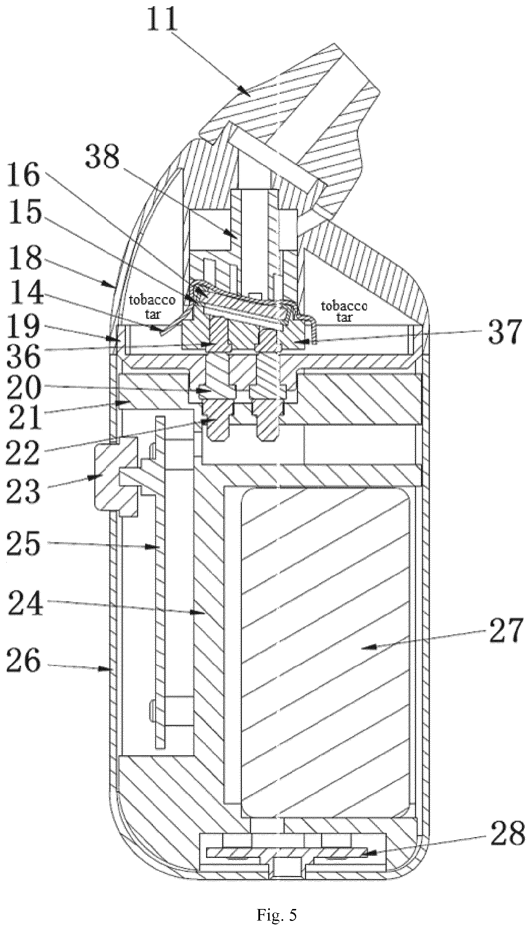

An electronic cigarette, as shown in Fig. 5, includes the above atomizer 1 and a battery assembly 2, wherein the atomizer 1 is magnetically connected with the battery assembly 2 to facilitate disassembly.

The battery assembly of the present teachings includes a battery cover 26, a large bracket 25, and a battery 27. A heat insulation space 29 is formed in the large bracket 25 to prevent the heat generated by the battery assembly 2 from being transferred to the battery cover 26 to scald hands when the electronic cigarette is in operation.

An upper fixing cover 19 is arranged at the bottom of the housing 18, a lower fixing cover 21 is arranged at the top of the battery cover 26, an upper electrode 20 is mounted on the upper fixing cover 19, a lower electrode 22 is mounted on the lower fixing cover 21, and the upper electrode 20 is electrically connected with the lower electrode 22. A key 23 is mounted on a side face of the battery cover 26, a PCB 24 is arranged inside the key 23, the battery 27 is electrically connected with the PCB 24, and the PCB 24 is in contact with the lower electrode 22 when the PCB 24 is pressed by the key 23, so that the battery 27 supplies power to the piezoelectric ceramic being as an ultrasonic atomizing sheet for ultrasonic atomization.

The present embodiment differs from Embodiment 1 in that:
1. The atomization cotton 14 is in direct contact with the tobacco tar, so that when the atomizer 1 is in operation, the tobacco tar guide rate is higher and the atomization effect is better.
2. The piezoelectric ceramic 16 is arranged in the tobacco tar cavity, and the piezoelectric ceramic 16 is placed obliquely. When the piezoelectric ceramic 16 is in operation, the smoke formed by atomization of the tobacco tar is jetted to the inner wall of the air outlet 39, the inner wall collects and condenses large-particle smoke into smoke droplets, and the smoke droplets drop onto the atomization cotton 14 along the inner wall and are re-atomized. During this process, small-particle smoke is sucked by the user, thus prevent the large-particle smoke from being sucked to affect the taste of the smoke.
3. The support pillar 41 is arranged on the atomizing seat 37, and abuts against the atomization cotton 14 to prevent the atomization cotton 14 from being separated from the piezoelectric ceramic 16 to affect the atomization effect.

The contents illustrated by the above embodiments should be understood as these embodiments are merely used for illustrating the present teachings more clearly, rather than limiting the scope of the present invention. Various equivalent modifications can be made to the present teachings by those skilled in the art as long as they fall within the scope defined by the appended claims of the present application.

## Claims

1. An atomizer, comprising a housing (18) comprising a bottom, an atomization cotton (14) and an ultrasonic atomizing sheet (16), wherein the atomization cotton (14) and the ultrasonic atomizing sheet (16) are arranged in the housing (18), **characterized in that** the ultrasonic atomizing sheet (16) is obliquely arranged in the housing (18) such that the ultrasonic atomizing sheet (16) is at different heights on two sides, and two ends of the atomization cotton (14) are connected to tobacco tar in a tobacco tar cavity via tobacco tar guide cotton (17), or the two ends of the atomization cotton (14) arc directly provided in the tobacco tar cavity; and a lower surface of the atomization cotton (14), which faces the ultrasonic atomizing sheet (16), is in contact with an atomization surface of the ultrasonic atomizing sheet, so that the atomization cotton is arranged from a high side to a low side of the ultrasonic atomizing sheet (16) and excess tobacco tar on the atomizing sheet (16) is returned to the tobacco tar guide cotton (17) or the tobacco tar cavity through the atomization cotton (14) at the lower end of the inclined portion of the atomization sheet (16).

2. The atomizer according to claim 1, wherein the ultrasonic atomizing sheet is a piezoelectric ceramic (16) driven by an electric conductor to ultrasonically vibrate.

3. The atomizer according to claim 1, wherein the ultrasonic atomizing sheet has an inclination angle of A, wherein 0° <A<90° , more preferably 50°≤A≤75°.

4. The atomizer according to any one of claims 1-3, wherein the ultrasonic atomizing sheet is arranged in a mounting cavity (34) of the housing (18), and the mounting cavity (34) is obliquely arranged; preferably, the ultrasonic atomizing sheet is arranged in the mounting cavity (34) through a silica gel sleeve (15), the bottom of the mounting cavity (34) is provided with a penetration groove (35), and the lower part of the atomization cotton (14) penetrates through the penetration groove (35) and is connected to the tobacco tar guide cotton (17).

5. The atomizer according to claim 4, wherein a locking ring (12) fixedly connected with the housing (18) is arranged at the upper part of the tobacco tar guide cotton (17), an air tube (31) for communicating with a suction nozzle (11) is formed in the center of the locking ring (12), an air passing slot (32) is formed in the outer wall of the locking ring (12), and the air passing slot (32) is used for forming an air inflow passage between the outside and the atomization cotton (14).

6. The atomizer according to claim 5, wherein a gap is formed between the bottom of the air tube (31) of the locking ring (12) and the upper surface of the atomization cotton (14); preferably, the width of the gap is 0.2 to 5 mm; more preferably, the width of the gap is 0.5 to 2.5 mm.

7. The atomizer according to any one of claims 1-3, wherein a pressing structure which maintains contact between the atomization cotton (14) and the atomization surface of the ultrasonic atomizing sheet is arranged on the upper surface of the atomization cotton (14); preferably, the pressing structure is a pressing spring (13).

8. The atomizer according to any one of claims 1-3, wherein the ultrasonic atomizing sheet is arranged at the top of an atomizing seat (37), and a silica gel seat (38) is arranged on the upper surface of the ultrasonic atomizing sheet; an air outlet (39) is formed in the center of the silica gel seat (38), and an air inlet (40) is formed at the lower part of the silica gel seat (38); the air outlet (39) is used for communicating with the suction nozzle (11), and the air inlet (40) is used for forming an air inflow passage between the outside and the atomization cotton (14).

9. The atomizer according to claim 8, wherein the bottom of the silica gel seat (38) is provided with a ventilation groove (42) which is used for communicating the air outlet (39) with the air inflow passage.

10. The atomizer according to claim 8, wherein the two ends of the atomization cotton (14) pass through the gap between the atomizing seat (37) and the silica gel seat (38) and stretch into the tobacco tar cavity.

11. The atomizer according to any one of claims 1 to 10, further comprising a suction nozzle (11),
wherein a locking ring (12) fixedly connected with the housing (18) is arranged at the upper part of the tobacco tar guide cotton (17); the housing (18) and the locking ring (12) are detachably and fixedly connected, and the housing (18) and the suction nozzle (11) are also detachably and fixedly connected; preferably, the internal thread of the housing (18) is connected to the external thread of the locking ring (12), and the external thread of the housing (18) is connected to the internal thread of the suction nozzle (11),
wherein preferably an air tube (31) is formed in the center of the locking ring (12), and the air tube (31) and the hollow passage of the suction nozzle (11) form an air outflow passage; an air inlet slot (30) is formed between the housing (18) and the suction nozzle (11), an air passing slot (32) is formed in the outer wall surface of the locking ring (12), and the air inlet slot (30) and the air passing slot (32) form an air inflow passage which communicates with the air outflow passage.

12. The atomizer according to claim 11, wherein the housing (18) and the suction nozzle (11) are detachably and fixedly connected; preferably, the internal thread of the housing (18) is connected to the external thread of the suction nozzle (11),
wherein preferably a silica gel seat (38) and an atomizing seat (37) are arranged in the housing (18), and the ultrasonic atomizing sheet and the atomization cotton (14) are arranged between the silica gel seat (38) and the atomizing seat (37); an air outlet (39) is formed in the center of the silica gel seat (38), and an air inlet (40) is formed at the lower part of the silica gel seat (38); the air outlet (39) communicates with the suction nozzle (11) to form an air outflow passage, and the air inlet (40) and the ventilation groove (42) arranged at the bottom of the silica gel seat (38) form an air inflow passage which communicates with the air outflow passage.

13. An electronic cigarette, wherein the electronic cigarette comprises a battery assembly (2) and the atomizer (1) according to claim 11 or 12, wherein the battery assembly (2) is used for driving the ultrasonic atomizing sheet to ultrasonically vibrate through a battery.

14. The electronic cigarette according to claim 13, wherein the atomizer (1) is detachably and fixedly connected to the battery assembly (2), and preferably, the atomizer (1) is magnetically connected to the battery assembly (2).

15. The electronic cigarette according to claim 13, wherein the battery assembly (2) comprises a battery cover (26), a large bracket (25) and a battery (27); and a heat insulation space (29) is formed in the large bracket (25),
wherein preferably an upper fixing cover (19) is arranged at the bottom of the housing (18), a lower fixing cover (21) is arranged at the top of the battery cover (26), an upper electrode (20) is mounted on the upper fixing cover (19), a lower electrode (22) is mounted on the lower fixing cover (21), and the upper electrode (20) is electrically connected with the lower electrode (22); a key (23) is mounted on a side face of the battery cover (26), a PCB (24) is arranged inside the key (23), the battery (27) is electrically connected with the PCB (24), and the PCB (24) is in contact with the lower electrode (22) when the PCB (24) is pressed by the key (23).

## Patentansprüche

1. Zerstäuber, enthaltend ein Gehäuse (18) mit einem Boden, eine Zerstäubungswatte (14) und ein Ultraschall-Zerstäubungsblatt (16), wobei die Zerstäubungswatte (14) und das Ultraschall-Zerstäubungsblatt (16) im Gehäuse (18) angeordnet sind, **dadurch gekennzeichnet, dass** das Ultraschall-Zerstäubungsblatt (16) schräg im Gehäuse (18) angeordnet ist, so dass sich das Ultraschall-Zerstäubungsblatt (16) auf zwei Seiten in unterschiedlichen Höhen befindet, und zwei Enden der Zerstäubungswatte (14) über eine Tabakteer-Führungswatte (17) mit Tabakteer in einem Tabakteer-Hohlraum verbunden sind, oder die beiden Enden der Zerstäubungswatte (14) direkt in dem Tabakteer-Hohlraum bereitgestellt sind; und eine untere Fläche der Zerstäubungswatte (14), die dem Ultraschall-Zerstäubungsblatt (16) zugewandt ist, in Kontakt mit einer Zerstäubungsfläche des Ultraschall-Zerstäubungsblatts ist, so dass die Zerstäubungswatte von einer hohen Seite zu einer niedrigen Seite der Ultraschall-Zerstäubungsblatt (16) angeordnet ist und überschüssiger Tabakteer auf dem Zerstäubungsblatt (16) über die Zerstäubungswatte (14) am unteren Ende des geneigten Abschnitts des Zerstäubungsblatts (16) zu der Tabakteer-Führungswatte (17) oder zu dem Tabakteer-Hohlraum zurückgeführt wird.

2. Zerstäuber nach Anspruch 1, bei dem das Ultraschall-Zerstäubungsblatt eine piezoelektrische Keramik (16) ist, die von einem elektrischen Leiter angeregt wird, um mit Ultraschall zu schwingen.

3. Zerstäuber nach Anspruch 1, bei dem das Ultraschall-Zerstäubungsblatt einen Neigungswinkel A aufweist, wobei 0°<A<90°, vorzugsweise 50°≤A≤75°.

4. Zerstäuber nach einem der Ansprüche 1 bis 3, bei dem das Ultraschall-Zerstäubungsblatt in einem Montagehohlraum (34) des Gehäuses (18) angeordnet ist und der Montagehohlraum (34) schräg angeordnet ist; vorzugsweise ist das Ultraschall-Zerstäubungsblatt in dem Montagehohlraum (34) durch eine Silikagel-Hülse (15) angeordnet, der Boden des Montagehohlraums (34) ist mit einer Durchdringungsnut (35) versehen und der untere Teil der Zerstäubungswatte (14) verläuft durch die Durchdringungsnut (35) ist und mit der Tabakteer-Führungswatte (17) verbunden.

5. Zerstäuber nach Anspruch 4, bei dem ein fest mit dem Gehäuse (18) verbundener Sicherungsring (12) am oberen Teil der Tabakteer-Führungswatte (17) angeordnet ist, eine Luftröhre (31) zur Verbindung mit einer Saugdüse (11) in der Mitte des Sicherungsrings (12) ausgebildet ist, ein Luftdurchlassschlitz (32) in der Außenwand des Sicherungsrings (12) ausgebildet ist und der Luftdurchlassschlitz (32) dazu dient, einen Lufteinströmunskanal zwischen der Außenseite und der Zerstäubungswatte (14) zu bilden.

6. Zerstäuber nach Anspruch 5, bei dem zwischen dem Boden der Luftröhre (31) des Sicherungsrings (12) und der oberen Fläche der Zerstäubungswatte (14) ein Spalt gebildet ist; vorzugsweise beträgt die Breite des Spalts 0,2 bis 5 mm; noch bevorzugter beträgt die Breite des Spalts 0,5 bis 2,5 mm.

7. Zerstäuber nach einem der Ansprüche 1 bis 3, bei dem eine Andruckstruktur, die den Kontakt zwischen der Zerstäubungswatte (14) und der Zerstäubungsfläche des Ultraschall-Zerstäubungsblatts aufrechterhält, auf der oberen Fläche der Zerstäubungswatte (14) angeordnet ist; vorzugsweise ist die Andruckstruktur eine Andruckfeder (13).

8. Zerstäuber nach einem der Ansprüche 1 bis 3, bei dem das Ultraschall-Zerstäubungsblatt Oben auf einem Zerstäubungssitz (37) angeordnet ist und ein Silikagelsitz (38) auf der oberen Fläche des Ultraschall-Zerstäubungsblatts angeordnet ist; ein Luftauslass (39) in der Mitte des Silikagelsitzes (38) ausgebildet ist und ein Lufteinlass (40) am unteren Teil des Silikagelsitzes (38) ausgebildet ist; der Luftauslass (39) zur Verbindung mit der Saugdüse (11) dient und der Lufteinlass (40) zur Bildung eines Lufteinlasskanals zwischen der Außenseite und der Zerstäubungswatte (14) verwendet wird.

9. Zerstäuber nach Anspruch 8, bei dem der Boden des Silikagelsitzes (38) mit einer Belüftungsnut (42) versehen ist, die zur Kommunikation des Luftauslasses (39) mit dem Lufteinlasskanal verwendet wird.

10. Zerstäuber nach Anspruch 8, bei dem die beiden Enden der Zerstäubungswatte (14) durch den Spalt zwischen dem Zerstäubungssitz (37) und dem Silikagelsitz (38) hindurchverlaufen und sich in den Tabakteer-Hohlraum erstrecken.

11. Zerstäuber nach einem der Ansprüche 1 bis 10, ferner enthaltend eine Saugdüse (11), wobei ein fest mit dem Gehäuse (18) verbundener Sicherungsring (12) am oberen Teil der Tabakteer-Führungswatte (17) angeordnet ist; das Gehäuse (18) und der Sicherungsring (12) lösbar und fest miteinander verbunden sind, und das Gehäuse (18) und die Saugdüse (11) ebenfalls lösbar und fest miteinander verbunden sind; vorzugsweise ist das Innengewinde des Gehäuses (18) mit dem Außengewinde des Sicherungsrings (12) verbunden, und das Außengewinde des Gehäuses (18) ist mit dem Innengewinde der Saugdüse ( 11 ) verbunden, wobei vorzugsweise eine Luftröhre (31) in der Mitte des Sicherungsrings (12) ausgebildet ist und die Luftröhre und der hohle Durchgang der Saugdüse ( 11) einen Luftaustrittsdurchgang bilden; ein Lufteinlassschlitz (30) ist zwischen dem Gehäuse (18) und der Saugdüse (11) ausgebildet, ein Luftdurchlassschlitz (32) ist in der Außenwandfläche des Sicherungsrings (12) ausgebildet und der Lufteinlassschlitz (30) und der Luftdurchlassschlitz (32) bilden einen Lufteinlasskanal, der mit dem Luftauslasskanal kommuniziert.

12. Zerstäuber nach Anspruch 11, bei dem das Gehäuse (18) und die Saugdüse (11) lösbar und fest miteinander verbunden sind; vorzugsweise ist das Innengewinde des Gehäuses (18) mit dem Außengewinde der Saugdüse ( 11 ) verbunden, wobei vorzugsweise ein Silikagelsitz (38) und ein Zerstäubungssitz (37) in dem Gehäuse (18) angeordnet sind, und das Ultraschall-Zerstäubungsblatt und die Zerstäubungswatte (14) zwischen dem Silikagelsitz (38) und dem Zerstäubungssitz (37) angeordnet sind; ein Luftauslass (39) in der Mitte des Silikagelsitzes (38) ausgebildet ist und ein Lufteinlass (40) am unteren Teil des Silikagelsitzes (38) ausgebildet ist; der Luftauslass (39) mit der Saugdüse (11) verbunden ist, um einen Luftauslasskanal zu bilden, und der Lufteinlass (40) und die Belüftungsnut (42), die am Boden des Silikagelsitzes (38) angeordnet sind, einen Lufteinlasskanal bilden, der mit dem Luftauslasskanal kommuniziert.

13. Eine elektronische Zigarette, wobei die elektronische Zigarette eine Batteriebaugruppe (2) und den Zerstäuber (1) gemäß Anspruch 11 oder 12 umfasst, wobei die Batteriebaugruppe (2) dazu verwendet wird, das Ultraschall-Zerstäubungsblatt über eine Batterie in Ultraschallschwingungen zu versetzen.

14. Elektronische Zigarette nach Anspruch 13, bei der der Zerstäuber (1) abnehmbar und fest mit der Batteriebaugruppe (2) verbunden ist, und vorzugsweise der Zerstäuber (1) magnetisch mit der Batteriebaugruppe (2) verbunden ist.

15. Elektronische Zigarette nach Anspruch 13, bei der die Batteriebaugruppe (2) eine Batterieabdeckung (26), eine große Halterung (25) und eine Batterie (27) enthält; und ein Wärmeisolationsraum (29) in der großen Halterung (25) ausgebildet ist, wobei vorzugsweise eine obere Befestigungsabdeckung (19) am Boden des Gehäuses (18) angeordnet ist, eine untere Befestigungsabdeckung (21) oben auf der Batterieabdeckung (26) angeordnet ist, eine obere Elektrode (20) an der oberen Befestigungsabdeckung (19) angebracht ist, eine untere Elektrode (22) an der unteren Befestigungsabdeckung (21) angebracht ist und die obere Elektrode (20) elektrisch mit der unteren Elektrode (22) verbunden ist; eine Taste (23) an einer Seitenfläche der Batterieabdeckung (26) angebracht ist, eine Leiterplatte (24) im Inneren des Tasters (23) angeordnet ist, die Batterie (27) elektrisch mit der Leiterplatte (24) verbunden ist und die Leiterplatte (24) in Kontakt mit der unteren Elektrode (22) steht, wenn die Leiterplatte (24) durch den Taster (23) gedrückt wird.

## Revendications

1. Atomiseur, comprenant un boîtier (18) comprenant un fond, un coton d'atomisation (14) et une feuille d'atomisation ultrasonore (16), dans lequel le coton d'atomisation (14) et la feuille d'atomisation ultrasonore (16) sont disposés dans le boîtier (18), **caractérisé en ce que** la feuille d'atomisation ultrasonore (16) est disposée de manière oblique dans le boîtier (18) de telle sorte que la feuille d'atomisation ultrasonore (16) est à des hauteurs différentes sur deux côtés et deux extrémités du coton d'atomisation (14) sont reliées à du goudron de tabac dans une cavité à goudron de tabac par l'intermédiaire d'un coton guide de goudron de tabac (17) ou les deux extrémités du coton d'atomisation (14) sont directement prévues dans la cavité à goudron de tabac ; et une surface inférieure du coton d'atomisation (14), qui fait face à la feuille d'atomisation ultrasonore (16), est en contact avec une surface d'atomisation de la feuille d'atomisation ultrasonore, de sorte que le coton d'atomisation est disposé d'un côté haut vers un côté bas de la feuille d'atomisation ultrasonore (16) et un excédent de goudron de tabac sur la feuille d'atomisation (16) est renvoyé au coton guide de goudron de tabac (17) ou la cavité à goudron de tabac à travers le coton d'atomisation (14) au niveau de l'extrémité inférieure de la section inclinée de la feuille d'atomisation (16).

2. Atomiseur selon la revendication 1, dans lequel la feuille d'atomisation ultrasonore est une céramique piézoélectrique (16) entraînée par un conducteur électrique pour vibrer de manière ultrasonore.

3. Atomiseur selon la revendication 1, dans lequel la feuille d'atomisation ultrasonore présente un angle d'inclinaison de A, dans lequel 0° < A < 90°, plus préférablement 50° ≤ A ≤ 75°.

4. Atomiseur selon l'une quelconque des revendications 1 à 3, dans lequel la feuille d'atomisation est disposée dans une cavité de montage (34) du boîtier (18) et la cavité de montage (34) est disposée de manière oblique ; de préférence, la feuille d'atomisation ultrasonore est disposée dans la cavité de montage (34) à travers un manchon de gel de silice (15), le fond de la cavité de montage (34) est doté d'une rainure de pénétration (35) et la partie inférieure du coton d'atomisation (14) pénètre à travers la rainure de pénétration (35) et est reliée au coton guide de goudron de tabac (17).

5. Atomiseur selon la revendication 4, dans lequel une bague de verrouillage (12) reliée de manière fixe au boîtier (18) est disposée au niveau de la partie supérieure du coton guide de goudron de tabac (17), un tube à air (31) destiné à communiquer avec une buse d'aspiration (11) est formé dans le centre de la bague de verrouillage (12), une fente de passage d'air (32) est formée dans la paroi externe de la bague de verrouillage (12) et la fente de passage d'air (32) est utilisée pour former un passage d'entrée d'air entre l'extérieur et le coton d'atomisation (14).

6. Atomiseur selon la revendication 5, dans lequel un espace est formé entre le fond du tube à air (31) de la bague de verrouillage (12) et la surface supérieure du coton d'atomisation (14) ; de préférence, la largeur de l'espace est de 0,2 à 5 mm ; plus préférablement, la largeur de l'espace est de 0,5 à 2,5 mm.

7. Atomiseur selon l'une quelconque des revendications 1 à 3, dans lequel une structure de pression qui maintient un contact entre le coton d'atomisation (14) et la surface d'atomisation de la feuille d'atomisation ultrasonore est disposée sur la surface supérieure du coton d'atomisation (14) ; de préférence, la structure de pression est un ressort de pression (13).

8. Atomiseur selon l'une quelconque des revendications 1 à 3, dans lequel la feuille d'atomisation ultrasonore est disposée au niveau du dessus d'un siège d'atomisation (37) et un siège de gel de silice (38) est disposé sur la surface supérieure de la feuille d'atomisation ultrasonore ; une sortie d'air (39) est formée dans le centre du siège de gel de silice (38) et une entrée d'air (40) est formée au niveau de la partie inférieure du siège de gel de silice (38) ; la sortie d'air (39) est utilisée pour communiquer avec la buse d'aspiration (11) et l'entrée d'air (40) est utilisée pour former un passage de flux d'air entre l'extérieur et le coton d'atomisation (14).

9. Atomiseur selon la revendication 8, dans lequel le fond du siège de gel de silice (38) est doté d'une rainure de ventilation (42) qui est utilisée pour faire communiquer la sortie d'air (39) avec le passage d'entrée d'air.

10. Atomiseur selon la revendication 8, dans lequel les deux extrémités du coton d'atomisation (14) passent à travers l'espace entre le siège d'atomisation (37) et le siège de gel de silice (38) et s'étirent dans la cavité de goudron de tabac.

11. Atomiseur selon l'une quelconque des revendications 1 à 10, comprenant en outre une buse d'aspiration (11),
dans lequel une bague de verrouillage (12) reliée de manière fixe au boîtier (18) est agencée au niveau de la partie supérieure du coton guide de goudron de tabac (17) ; le boîtier (18) et la bague de verrouillage (12) sont reliés de manière détachable et fixe et le boîtier (18) et la buse d'aspiration (11) sont également reliés de manière détachable et fixe ; de préférence, le filetage interne du boîtier (18) est relié au filetage externe de la bague de verrouillage (12) et le filetage externe du boîtier (18) est relié au filetage interne de la buse d'aspiration (11),
dans lequel de préférence un tube à air (31) est formé dans le centre de la bague de verrouillage (12) et le tube à air (31) et le passage creux de la buse d'aspiration (11) forment un passage de sortie d'air ; une fente d'entrée d'air (30) est formée entre le boîtier (18) et la buse d'aspiration (11), une fente de passage d'air (32) est formée dans la surface de paroi externe de la bague de verrouillage (12) et la fente d'entrée d'air (30) et la fente de passage d'air (32) forment un passage d'entrée d'air qui communique avec le passage de sortie d'air.

12. Atomiseur selon la revendication 11, dans lequel le boîtier (18) et la buse d'aspiration (11) sont reliés de manière détachable et fixe ; de préférence, le filetage interne du boîtier (18) est relié au filetage externe de la buse d'aspiration (11),
dans lequel de préférence un siège de gel de silice (38) et un siège d'atomisation (37) sont disposés dans le boîtier (18) et la feuille d'atomisation ultrasonore et le coton d'atomisation (14) sont disposés entre le siège de gel de silice (38) et le siège d'atomisation (37) ; une sortie d'air (39) est formée dans le centre du siège de gel de silice (38) et une entrée d'air (40) est formée au niveau de la partie inférieure du siège de gel de silice (38) ; la sortie d'air (39) communique avec la buse d'aspiration (11) pour former un passage de sortie d'air et l'entrée d'air (40) et la rainure de ventilation (42) disposées au niveau du fond du siège de gel de silice (38) forment un passage d'entrée d'air qui communique avec le passage de sortie d'air.

13. Cigarette électronique, dans laquelle la cigarette électronique comprend un ensemble formant batterie (2) et l'atomiseur (1) selon la revendication 11 ou 12, dans lequel l'ensemble formant batterie (2) est utilisé pour entraîner la feuille d'atomisation ultrasonore pour qu'elle vibre de manière ultrasonore à travers une batterie.

14. Cigarette électronique selon la revendication 13, dans laquelle l'atomiseur (1) est relié de manière détachable et fixe à l'ensemble formant batterie (2) et, de préférence, l'atomiseur (1) est relié de manière magnétique à l'ensemble formant batterie (2).

15. Cigarette électronique selon la revendication 13, dans laquelle l'ensemble formant batterie (2) comprend un couvercle de batterie (26), un grand support (25) et une batterie (27) ; et un espace d'isolation thermique (29) est formé dans la grand support (25),
dans lequel de préférence un couvercle de fixation supérieure (19) est disposé au niveau du fond du boîtier (18), un couvercle de fixation inférieure (21) est disposé au niveau de la partie supérieure du couvercle de batterie (26), une électrode supérieure (20) est montée sur le couvercle de fixation supérieure (19), une électrode inférieure (22) est montée sur le couvercle de fixation inférieure (21) et l'électrode supérieure (20) est reliée électriquement à l'électrode inférieure (22) ; une bouton (23) est montée sur une face latérale du couvercle de batterie (26), une carte de circuit imprimé (24) est disposée à l'intérieur de la bouton (23), la batterie (27) est reliée électriquement à la carte de circuit imprimé (24) et la carte de circuit imprimé (24) est en contact avec l'électrode inférieure (22) lorsque la carte de circuit imprimé (24) est pressée par la bouton (23).
